Europäisches Patentamt

European Patent Office

Office européen des brevets

(19)

(11) Publication number: **0 393 299**
**A1**

## (12) EUROPEAN PATENT APPLICATION

(21) Application number: **89850123.4**

(51) Int. Cl.⁵: **A61L 2/08**

(22) Date of filing: **20.04.89**

| | |
|---|---|
| (43) Date of publication of application:<br>**24.10.90 Bulletin 90/43**<br><br>(84) Designated Contracting States:<br>**AT BE CH DE ES FR GB GR IT LI LU NL SE** | (71) Applicant: **TREMEDIC AB**<br>**Box 5**<br>**S-440 60 Skärhamn(SE)**<br><br>(72) Inventor: **Wideström, Hans Ake Lennart**<br>**Nygatan 9**<br>**S-440 60 Skärhamn(SE)**<br><br>(74) Representative: **Ryrlén, Evert**<br>**ALFONS HEDBERGS PATENTBYRÄ AB**<br>**Aschebergsgatan 35**<br>**S-411 33 Göteborg(SE)** |

(54) **Sterile gels.**

(57) A sterile water-base gel, primarily for medical purposes. The gel contains carboxypolymethylene allowing the gel to be sterilized by means of gamma radiation.

EP 0 393 299 A1

## Sterile Gels

Gels and ointments are widely used for medical purposes as lubricants, as vehicles for medically active substances and for various other purposes. Products of this kind must by necessity be sterile. The sterile property is imparted e.g. through steam-heating in autoclaves. Also aseptically packaged gels exist, usually in combination with preserving agents, such as benzoic acid, para-benzenes, parachloric methacresate. However the use of preserving agents is often unsuitable as such agents may affect the active substance and may induce allergies in sensitive persons.

Many modern packages made of plastic degrade when exposed to the heat prevailing in autoclaves. For this reason and because of the negative aspects of using preserving agents as indicated above it has been suggested to effect sterilization by means of ionizing radiation. Unfortunately, however, water-base gels based on gum arabic, tragacanth carbomer, carboxymethylene cellulose and other gelling agents lose their gel structure when exposed to ionizing radiation and form a low-viscosity acqueous solution.

However, it has been found that carbomer (carboxypolymethylene) may be stabilized with the aid of polyvalent alcohols, such as ethylene glycol, glycerine, propylene glycol which prevent the gel from degrading when sterilized e.g. when exposed to gamma radiation from Cobalt 60. See Table 1.

TABLE 1

| Carbopol 940 1 % | 250 g | 200 g | 200 g | 200 g |
|---|---|---|---|---|
| Sodium hydroxide | 0.04 g | 0.03 g | 0.1 g | 0.1 g |
| 2-methylene pentadiole | 150 g | - | - | - |
| Propylene glycol | - | 50 g | - | - |
| Glycerol | - | - | 200 g | - |
| Viscosity prior to radiation, in CP | 70,000 | 48,000 | 70,000 | 90,000 |
| Viscosity after radiation | 60,000 | 49,000 | 65,000 | 6 |

The effects as regards stability have been studied in closer details in glycerine, a substance possessing thoroughly documented cosmetic properties. The effects appear from Table 2.

TABLE 2

| Glycerine contents in % | 0 | 1 | 2,5 | 5 | 10 | 25 | 50 |
|---|---|---|---|---|---|---|---|
| 2 %-Carbopol 934 g | 200 | 200 | 200 | 200 | 200 | 200 | 200 |
| 99 %-Glycerol g | - | 4 | 10 | 20 | 40 | 100 | 200 |
| Distilled water g | 200 | 196 | 190 | 180 | 160 | 100 | - |
| Sodium Hydroxide g | 0,1 | 0,1 | 0,1 | 0,1 | 0,1 | 0,1 | 0,1 |
| Viscosity prior to $\gamma$, in CP | 41,000 | 42,000 | 43,000 | 55,000 | 54,000 | 78,000 | 72,000 |
| Viscosity after $\gamma$, in Cp | 3,400 | 29,000 | 37,000 | 50,000 | 45,000 | 60,000 | 65,000 |

As evidenced from Table 2, contents as low as a few percentages have a stabilizing effect on carboxypolymethylene gels.

## Claims

1. A sterile water-base gel, primarily for medical purposes, **characterized therein** that it contains carboxypolymethylene polymers (carbomer) and, as stabilizing agents, polyvalent alcohols, and in that it is sterilized by means of ionizing radiation.

2. A gel as claimed in claim 1, **characterized therein** that it contains from 1 to 90 % stabilizing polyvalent alcohols.

European Patent
Office

**EUROPEAN SEARCH REPORT**

Application Number

EP 89 85 0123

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 79, no. 26, 31st December 1973, page 181, abstract no. 149311r, Columbus, Ohio, US; I. ADAMS et al.: "Formulation and sterilization of a surgical lubricant gel based on carboxypolymethylene", & J. PHARM. PHARMACOL. 1973, 25(8), 640-6 * Abstract * | 1 | A 61 L 2/08 |

TECHNICAL FIELDS
SEARCHED (Int. Cl.5)

A 61 L

The present search report has been drawn up for all claims

| Place of search | Date of completion of the search | Examiner |
|---|---|---|
| THE HAGUE | 21-11-1989 | PELTRE CHR. |

EPO FORM 1503 03.82 (P0401)